**Europäisches Patentamt**

**European Patent Office**

**Office européen des brevets**

(19)

(11) Numéro de publication: **0 116 579**

**B1**

# FASCICULE DE BREVET EUROPÉEN

(12)

(45) Date de publication du fascicule du brevet:
10.05.89

(21) Numéro de dépôt: 83902530.1

(22) Date de dépôt: 28.07.83

(86) Numéro de dépôt international:
PCT/FR 83/00159

(87) Numéro de publication internationale:
WO 84/00491 (16.02.84 Gazette 84/05)

(51) Int. Cl.⁴: **A 61 K 35/32,** A 61 K 35/48,
A 61 K 35/54, A 61 K 35/12,
A 61 K 37/12

(54) **L'IMPLANT ANTI-RIDE.**

(30) Priorité: 30.07.82 FR 8213367

(43) Date de publication de la demande:
29.08.84 Bulletin 84/35

(45) Mention de la délivrance du brevet:
10.05.89 Bulletin 89/19

(84) Etats contractants désignés:
AT BE CH DE FR GB LI LU NL SE

(56) Documents cité:
BE-A-648 992
DE-B-1 302 401
FR-A-1 355 783
FR-A-2 413 912
US-A-3 551 560

Le dossier contient des informations techniques
présentées postérieurement au dépôt de la
demande et ne figurant pas dans le présent
fascicule.

(73) Titulaire: HECMATI, Michel, 63, boulevard de la
Croisette, F-06400 Cannes (FR)

(72) Inventeur: HECMATI, Michel, 63, boulevard de la
Croisette, F-06400 Cannes (FR)

(74) Mandataire: Hautier, Jean- Louis, Cabinet Hautier
Office Méditerranéen de Brevets d'Invention et
de Marques 24 rue Masséna, F-06000 Nice (FR)

Il est rappelé que: Dans un délai de neuf mois à compter de la date de publication de la mention de la délivrance du brevet européen
toute personne peut faire opposition au brevet européen délivré, auprès de l'Office européen des brevets. L'opposition doit être
formée par écrit et motivée. Elle n'est réputée formée qu'après paiement de la taxe d'opposition (Art. 99(1) Convention sur le brevet
européen).

2

## Description

La présente invention concerne de manière générale la chirurgie esthétique et plus spécialement un implant anti-ride.

Il est bien connu que la peau du visage et de façon plus générale, les régions cutanées qui ne sont pas protégées par les vêtements subissent des altérations plus ou moins profondes dues aux agents physiques extérieurs, comme l'état hygrométrique de l'atmosphère et la lumière solaire directe. A ces rides d'origine extérieure s'ajoutent, pour le visage, celles dues à l'activité musculaire, comme les rides frontales ou les rides à la commissure des paupières. Bien que les rides soient sans importance physiologique, elles peuvent se révéler psychologiqueme insupportables par l'image d'une décrépitude précoce perçue par le sujet. L'effacement des rides est par conséquent resté au centre des préoccupations depuis les temps les plus anciens.

Parmi les traitements classiques des rides, si l'on fait abstraction des moyens cosmétiques qui sont essentiellement palliatifs, il convient de citer les interventions de la chirurgie esthétique cutanée qui permettent, il est vrai, des restaurations après certaines altérations graves, mais dont les inconvénients évidents sont de laisser subsister des cicatrices parfois difficiles à dissimuler et de modifier les traits. Le domaine particulier de la greffe cutanée à suscité les recherches les plus diverses qui n'ont pas offert de solutions parfaitement satisfaisantes non plus. En effet, aux inconvénients de la cicatrisation, qui est elle-même à envisager comme étant une source de rides, s'ajoutent ceux propres à l'hétéroplastie et à l'homoplastie. L'hétéroplastie ou hétérogreffe, qui est pratiquée avec du tissu provenant d'une espèce animale différente de celle du sujet, conduit inéluctablement à des réactions de rejet, mais ces dernières ne sont pas absentes non plus après homoplastie ou homogreffe, c'est-à-dire au moyen d'un tissu dont le donneur appartient à la même espèce que le sujet. De façon évidente, l'autogreffe, effectuée avec du tissu prélevé sur le sujet lui-même, est seule à échapper à cette critique, mais la cicatrice de prélèvement constitue un obstacle décisif pour une intervention de ce genre.

Parallèlement aux techniques de la greffe, celles de l'implantation ont aussi donné lieu à des investigations détaillées en chirurgie plastique. Ainsi, des implants aussi divers que le placenta, les silicones, la paraffine, les métaux comme l'or ou le magnésium, certaines vitamines et graisses et la peau, outre, très récemment le collagène, ont été mis en oeuvre. Les inconvénients sont apparus nombreux réactions de rejet ou d'allergie, efficacité limitée à un territoire restreint (par exemple intradermique mais non sous-cutanée).

Néanmoins, les recherches conduites par le Dr HECHMATI Michel, demandeur, dans le domaine des implants l'ont amené, compte tenu des résultats acquis par lui lors de l'implantation de cartilage animal depuis 15 ans, à la découverte d'un implant anti-ride donnant parfaite satisfaction.

Conformément à l'invention, on évite tous les inconvénients des traitements anti-rides classiques par l'implantation d'un tissu hétérologue non allergissant polymorphe, c'est-à-dire plastique.

L'invention consiste en un collagène de cartilage qui a été prélevé sur un foetus de ruminant et qui a subi uniquement un traitement de macération et de malaxation, la macération ayant compris une immersion de 25 à 35 jours des tissus cartilagineux dans un mélange de volumes égaux de sérum physiologique à 9 % de type classique et d'éthanol à 90 %, les tissus cartilagineux ayant été rincés dans du sérum physiologique à 9 % la malaxation ayant consisté à malaxer les cartilages dans un broyeur pour obtenir un mélange homogène injectable.

Le tissu hétérologue non allergissant polymorphe utilisé aux fins de l'invention est du cartilage, sans caractère de biogène, qui a été prélevé sur un foetus animal ou sur un jeune animal en cours de croissance et qui a subi un traitement dont les effets sont triples, à savoir disparition de tout caractère allergique, stabilité à la conservation et résistance illimitée à la résorption dans l'organisme du sujet traité.

L'expérience a révélé que les espèces animales convenant le mieux pour la collecte du collagène appartiennent aux bovidés et aux ovidés et sont spécialement le veau et le mouton. L'animal ou le foetus animal est disséqué en atmosphère stérilisante contenant de l'ozone pour la sélection du tissu cartilagineux. Les cartilages ainsi recueillis sont conditionnés ensuite, toujours sous atmosphère ozonisée, pour conservation pendant 30 jours dans un mélange de parties égales en volume de sérum physiologique à 9 pour mille de type habituel et d'éthanol à 90 pour cent. Après le trentième jour de conditionnement en milieu hydro-alcoolique sensiblement isotonique, les cartilages sont rincés au sérum physiologique jusqu'à épuisement de l'alcool, subdivisés si leur dimension l'exige, malaxés jusqu'à l'obtention d'un mélange homogène injectable, et mis en seringue sous la forme d'un mélange de 90 pour cent de cartilage et de 10 pour cent de sérum physiologique à 9 pour mille. Toutes ces opérations sont exécutées en atmosphère ozonisée et à la température ambiante.

Le cartilage prêt à l'injection présencé en seringue stérile peut être conservé pendant 3 à 6 mois avant l'usage, de préférence à une température de 9 à 12°C.

Le produit ainsi obtenu constitue un implant non résorbable qui fait disparaître complètement les rides sans provoquer aucune réaction de rejet ou d'allergie.

Dans la pratique, le traitement est effectué soit par injection au moyen de la seringue dans laquelle le produit est conditionné, soit par incision suivant une ligne de Langer.

En raison du prélèvement aseptique et du conditionnement à l'aide de milieux dénués de tout pouvoir irritant, l'implant conforme à l'invention, quoiqu'il ne soit nullement résorbé par l'organisme et soit donc parfaitement durable, ne provoque aucune réaction de rejet, au contraire de certains dérivés du collagène actuellement commercialisés dont les contre-indications sont multiples, par exemple des éruptions cutanées, le goître exophtalmique, la maladie de Crohn ou la colite ulcéreuse, pour n'en citer que quelque-uns. La raison des nombreuses contre-indications des collagènes actuellement déjà commercialisés est qu'ils contiennent certaines substances chimiques, comme la lidocaïne, entre autres. L'hétérogénéité de collecte de certains des produits du commerce pourrait être une raison pour laquelle ils ne se prêtent pas à une application aisée et sans réactions secondaires.

Au cours d'un certain nombre d'expériences préalables sur des sujets volontaires, le traitement a été effectué avec succès par voie intradermique, sous-cutanée ou plus profonde contre les rides du rictus, les ridules de la bouche et les pattes d'oie, de même que pour la correction du profil des pommettes ou du menton.

Pour la pratique courante du traitement d'une ride du visage, il suffit de nettoyer la peau à l'alcool et de désensibiliser la région à traiter par refroidissement au moyen d'un glaçon passé sur le visage. Les injections sont ensuite réalisées tout au long de la ride suivant la profondeur de celle-ci et sont suivies d'un massage du cartilage sous la peau avec les doigts. Le traitement peut être effectué en une ou deux séances et peut être répété, si la chose apparaît nécessaire, bien qu'il en soit rarement ainsi.

**Revendications**

1. Implant tissulaire naturel pour la mise en oeuvre d'un traitement anti-rides caractérisé par le fait

qu'il consiste en un collagène de cartilage qui a été prélevé sur un foetus de ruminant et qui a subi uniquement un traitement de macération et de malaxation, la macération ayant compris une immersion de 25 à 35 jours des tissus cartilagineux dans un mélange de volumes égaux de sérum physiologique à 9 % de type classique et d'éthanol à 90 %, les tissus cartilagineux ayant été rincés dans du sérum physiologique à 9 % la malaxation ayant cinsisté à malaxer les cartilages dans un broyeur pour obtenir un mélange homogène injectable.

2. Implant selon la revendication 1 caractérisé par le fait que le ruminant est la vache.

3. Implant selon la revendication 1 caractérisé par le fait que le ruminant est la brebis.

4. Procédé de fabrication d'un implant tissulaire naturel pour la mise en oeuvre d'un traitement anti-rides caractérisé par le fait que l'on prélève sur un foetus de ruminant les tissus cartilagineux de celui-ci, on met les tissus cartilagineux à macérer pendant 25 à 35 jours par immersion dans un mélange de volumes égaux de solution physiologique à 9 % de type classique et d'éthanol à 90 % on rince exhautivement le tissu cartilagineux dans du sérum physiologique à 9 % de type classique, on malaxe ensuite les cartilages dans un broyeur pour obtenir un mélange homogène injectable, et on introduit le cartilage ainsi traité dans un emballage scellé stérile, ces différentes opérations étant exécutées en atmosphère ozonisée et à la température ambiante.

**Patentansprüche**

1. Natürliches Gewebeimplantat zur Durchführung einer Antifaltenbehandlung, gekennzeichnet durch, ein Knorpelcollagen, das dem Fötus eines Wiederkäuers entnommen und nur einer Laug- und Misch- und Knetbehandlung unterworfen wurde, wobei das Laugen ein 25- bis 35-tägiges Eintauchen der Knorpelgewebe in ein Gemisch aus gleichen Volumina 9 %-igen klassischen physiologischen Serums und 90 % Ethanols umfaßt, die Knorpelgewebe mit 9 % physiologischem Serum gewaschen und in einem Mischer zu einem homogenen injizierbaren Gemisch gemischt wurden.

2. Implantat nach Anspruch 1, dadurch gekennzeichnet, daß der Wiederkäuer eine Kuh ist.

3. Implantat nach Anspruch 1, dadurch gekennzeichnet, daß der Wiederkäuer ein Mutterschaf ist.

4. Verfahren zur Herstellung eines natürlichen Gewebeimplantats zur Durchführung einer Antifaltenbehandlung, dadurch gekennzeichnet, daß dem Fötus eines Wiederkäuers die Knorpelgewebe entnommen werden, die 25 bis 35 Tage durch Eintauchen in ein Gemisch aus gleichen Volumina einer 9 %-igen klassischen physiologischen Lösung und 90 % Ethanol ausgelaugt, ausgiebig in 9 %-igem klassischem physiologischem Serum gewaschen, dann in einer Mühle zu einem homogenen injizierbaren Gemisch vermischt und geknetet werden und der so hergestellte Knorpel in eine sterile und verschlossene Verpackung eingeführt wird, wobei diese verschiedenen Maßnahmen in einer Ozonatmosphäre bei Raumtemperatur durchgeführt werden.

**Claims**

1. Natural tissue implant for carrying out an anti-wrinkle treatment, characterized in that it consists of a cartilage collagen having been taken in a fetus of a ruminant and having only undergone a maceration and malaxage treatment, the maceration comprising an immersion of the cartilage tissues in a mixture of equal volumes of 9 % physiological classical serum and 90 % ethanol for 25 to 35 days, the cartilage tissues having been rinced in 9 % physiological serum and malaxed in a mill to a homogeneous injectable mixture.

2. Implant according to claim 1, characterized in that the ruminant is a cow.

3. Implant according to claim 1, characterized in that the ruminant is an ewe.

4. Process for producing a natural tissue implant for carrying out an anti-wrinkle treatment, characterized in that the cartilage tissues are taken in a fetus of a ruminant, macerated for 25 to 35 days by immersion in a mixture of equal volumes of a 9 % classical physiological solution and 90 % ethanol, rinces exhaustively in 9 % classical physiological serum, then malaxed in a mill to a homogeneous injectable mixture and introduced into a sterile and sealed package, these different operations being carried out in an ozone atmosphere at room temperature.